# EUROPEAN PATENT APPLICATION

(11) **EP 1 935 415 A2**
(43) Date of publication of application: **25.06.2008**
(21) Application number: 08153021.4
(22) Date of filing: 19.09.2005
(51) Int. Cl.: A61K 31/138, A61P 5/26

(54) **Treatment of androgen-deprivation induced osteoporosis**

(30) Priority: 20.09.2004 US 944465
(62) Divisional of application: 05797480.0
(71) Applicant: GTX, Inc., Memphis, TN 38163 (US)
(72) Inventor: Steiner, Mitchell S., Germantown, TN 38139 (US); Veverka, Karen A., Cordova, TN 38018 (US); Raghow, Sharan, Collierville, TN 38017 (US)
(74) Representative: Vossius & Partner

(57) **Abstract**

The present invention provides methods for reducing the incidence of inhibiting, suppressing, and treating androgen-deprivation induced osteoporosis, bone fractures and/or loss of bone mineral density (BMD) in men having prostate cancer, comprising administering to a male human subject having prostate cancer a toremifene and/or its analog, derivative, isomer, metabolite, pharmaceutically acceptable salt, pharmaceutical product, hydrate, N-oxide or any combination thereof. The present invention also provides methods of treating, preventing, suppressing, inhibiting, or reducing the incidence of hot flashes, gynecomastia, and/or hair loss in a subject, comprising same.

## Description

The present invention provides methods for reducing the incidence of, inhibiting, suppressing, reducing the incidence of, and treating androgen-deprivation induced osteoporosis, bone fractures and/or loss of bone mineral density (BMD) in a subject, comprising administering to a subject a toremifene and/or its analog, derivative, isomer, metabolite; pharmaceutically acceptable salt, pharmaceutical product, hydrate, N-oxide, or any combination thereof. The present invention also provides methods of treating, preventing, suppressing, inhibiting, or reducing the incidence of hot flashes, gynecomastia, and/or hair loss in subjects, comprising same.

It is well established that the bone mineral density of males decrease with age. Decreased amounts of bone mineral content and density correlate with decreased bone strength and predispose to fracture. The molecular mechanisms underlying the pleiotropic effects of sex hormones in non-reproductive tissues are only beginning to be understood, but it is clear that physiologic concentrations of androgens and estrogens play an important role in maintaining bone homeostasis throughout the life cycle. Consequently, when androgen or estrogen deprivation occurs, there is a resultant increase in the rate ofbone remodeling that tilts the balance of resorption and formation in the favor of resorption, contributing to an overall loss of bone mass. In males, the natural decline in sex hormones at maturity (direct decline in androgens as well as lower levels of estrogens derived from peripheral aromatization of androgens) is associated with the frailty of bones. This effect is also observed in males who have been castrated.

Prostate cancer is one of the most frequently diagnosed noncutaneous cancers among men in the United States. One of the approaches to the treatment of prostate cancer is by androgen deprivation. The male sex hormone, testosterone, stimulates the growth of cancerous prostatic cells and, therefore, is the primary fuel for the growth of prostate cancer. The goal of androgen deprivation is to decrease the stimulation by testosterone of the cancerous prostatic cells. Testosterone normally is produced by the testes in response to stimulation from a hormonal signal called luteinizing hormone (LH), which in turn is stimulated by luteinizing-harmone releasing hormone (LH-RH). Androgen deprivation is accomplished either surgically by bilateral orchidectomy or chemically by LH-RH agonists (LHRH) with or without nonsteroidal anti-androgens.

Current studies suggest that early androgen deprivation in patients with micrometastatic disease may indeed prolong survival [Messing EM, et al (1999), N Engl J Med 34, 1781-1788; Newling (2001), Urology 58(Suppl 2A), 50-55]. Moreover, androgen deprivation is being employed in numerous new clinical settings, including neoadjuvant therapy prior to radical prostatectomy, long-term adjuvant therapy for patients at high incidence for recurrence following radiation or surgery, neoadjuvant therapy for radiation, and treatment of biochemical recurrence following radiation or surgery [Carroll, et al (2001), Urology 58, 1-4; Horwitz EM, et al (2001), Int J Radiat Oncol Biol Phy Mar 15;49(4), 947-56]. Thus, more prostate cancer patients have become candidates for and are being treated by androgen ablation. Moreover, these prostate cancer patients are being treated earlier and longer than in the past, which in some cases may be as long as 10 or more years of androgen deprivation therapy (ADT).

Unfortunately, ADT is fraught with significant side effects, including hot flashes, gynecomastia, osteoporosis, decreased lean muscle mass, depression and other mood changes, loss of libido, and erectile dysfunction [Stege R (2000), Prostate Suppl 10,38-42]. Consequently, complications of androgen blockade now contribute significantly to the morbidity, and in some cases the mortality, of men suffering from prostate cancer.

Given that more patients today are being treated by long-term androgen deprivation, osteoporosis has become a clinically important side effect in men suffering from prostate cancer undergoing androgen deprivation. Loss of bone mineral density (BMD) occurs in the majority of patients being treated by androgen deprivation by 6 months. New innovative approaches are urgently needed at both the basic science and clinical levels to decrease the incidence of androgen-deprivation induced osteoporosis in men suffering from prostate cancer.

This invention relates to a method of treating androgen-deprivation induced osteoporosis in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method of preventing androgen-deprivation induced osteoporosis in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof

This invention relates to a method of suppressing or inhibiting androgen-deprivation induced osteoporosis in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method of reducing the incidence of androgen-deprivation induced osteoporosis in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method of treating androgen-deprivation induced loss of BMD in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method ofpreventing androgen-deprivation induced loss of BMD in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method of suppressing or inhibiting androgen-deprivation induced loss of BMD in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method of reducing the incidence of androgen-deprivation induced loss of BMD in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method of treating androgen-deprivation induced bone fractures in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method of preventing androgen-deprivation induced bone fractures in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method of suppressing or inhibiting androgen-deprivation induced bone fractures in amale subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

This invention relates to a method of reducing the incidence of androgen-deprivation induced bone fractures in a male subject having prostate cancer, the method comprising the step of administering to said subject an anti-estrogen agent and/or its - analog, derivative, isomer, metabolite, pharmaceutically acceptable salt, pharmaceutical product, hydrate, N-oxide, or any combination thereof.

In another embodiment, the present invention provides a method of treating hot flashes in subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of suppressing or inhibiting hot flashes in a subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of reducing the incidence ofhot flashes in a subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of treating gynecomastia in a male subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of suppressing or inhibiting gynecomastia in a male subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of reducing the incidence of gynecomastia in a male subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of treating hair loss in a subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of suppressing or inhibiting hair loss in a subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of reducing the incidence of hair loss in a subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

The present invention will be understood and appreciated more fully from the following detailed description taken in conjunction with the appended figures which depict

Figure 1: Effect of toremifene on prostate weight in the Orx rat.

Figure 2a: Effect of toremifene on Osteocalin in rat serum.

Figure 2b: Effect of toremifene on RatLaps in rat serum.

Figure 3: Effect of toremifene on BMD in the ORX Rat.

Figure 4: Effect of toremifene on BMD in patients receiving ADT.

Figure 5: Effect of toremifene on bone turnover markers in patients receiving ADT. "*" indicates p < 0.05 relative to placebo.

Figure 6: Effect of toremifene on bone resorption markers in patients receiving ADT. *: p = 0.01; **: p = 0.05; ***: p = 0.38. BAP = bone alkaline phosphatase. U-CTX = urinary C telopeptide. U-NTX = urinary N telopeptide.

Figure 7: Effect of toremifene on pituitary hormone levels in patients receiving ADT. LH= Luteinizing hormone. FSH= Follicle stimulating hormone.

In another embodiment, this invention provides: 1) a method of treating androgen-deprivation induced osteoporosis in a male subject having prostate cancer; 2) a method of preventing androgen-deprivation induced osteoporosis in a male subject having prostate cancer, 3) a method of suppressing or inhibiting androgen-deprivation induced osteoporosis in a male subject having prostate cancer; 4) a method of reducing the incidence of androgen-deprivation induced osteoporosis in a male subject having prostate cancer, 5) a method of treating androgen-deprivation induced loss ofBMD in a male subject having prostate cancer; 6) a method of preventing androgen-deprivation induced loss of BMD in a male subject having prostate cancer; 7) a method of suppressing or inhibiting androgen-deprivation induced loss af BMD in a male subject having prostate cancer; 8) a method of reducing the incidence of androgen-deprivation induced loss ofBMD in a male subject having prostate cancer; 9) a method oftreating androgen-deprivation induced bone fractures in a male subject having prostate cancer; 10) a method of preventing androgen-deprivation induced bone fractures in a male subject having prostate cancer; 11) a method of suppressing or inhibiting androgen-deprivation induced bone fractures in a male subject having prostate cancer; 12) a method of reducing the incidence of androgen-deprivation induced bone fractures in a male subject having prostate cancer by administering to the subject an anti-estrogen agent and/or its analog, derivative, isomer, metabolite, pharmaceutically acceptable salt, pharmaceutical product, hydrate, N-oxide, or any combination thereof.

It is to be understood that a method of treating, reducing the incidence of, suppressing, or inhibiting a disease, condition or disorder in the present invention may refer, in one embodiment, to the use of a compound in the preparation of a pharmaceutical composition for use in the treatment of a disease, condition or disorder.

In one embodiment, the anti-estrogen that treats, prevents, suppresses, inhibits or reduces the incidence of androgen-deprivation induced osteoporosis and/or loss of BMD is a selective estrogen receptor modulator (SERM) and/or its analog, derivative, isomer, metabolite, pharmaceutically acceptable salt, pharmaceutical product, hydrate, N-oxide, or any combination thereof.

In another embodiment, the term "anti-estrogen" refers to compounds that antagonize the release or action of estrogens. Antiestrogens are known in the art (e.g., tamoxifen and derivatives thereof, such as trioxifene, toremifene and droloxifene) and are commercially available (e.g., tamoxifen; trade name: NOLVADEX.TM., a product of ICI Pharmaceuticals).

In one embodiment, the SERMs that are encompassed by the present invention include, but are not limited to, the following embodiments: triphenylalkylenes such as triphenylethylenes, which include Tamoxifen, Droloxifene, toremifene, Idoxifene, Clomiphene, Enclomiphene and Zuclomiphene; benzothiphene derivatives such as Raloxifene and LY 353381; benzopyran derivatives such as EM 800 (SCH 57050) and its metabolite EM 652; naphthalene derivatives such as Lasofoxifene (CP 336,156); chromans such as Levormeloxifene or their analogs, derivatives, isomers, or metabolites thereof, or their pharmaceutically acceptable salts, esters, N oxides, or mixtures thereof.

As contemplated herein, other embodiments of anti-estrogens that are encompassed by the present invention include but are in no way limited to the following embodiments: Cycladiene, Merck Index, 10th ed. #3085 and U.S. Pat. No. 2,464,203 and U.S.Pat.No. 2,465,505; Nafoxidine, USAN and USP Dictionary ofDrugNames, p. 327 (1983); CI-680, Unlisted Drugs, 28(10): 169(o) (1976); CI-628, Unlisted Drugs, 26(7): 106(1) (1974); CN-55,945-27, or nitromifene citrate, Unlisted Drugs, 27(12): 194(n) (1975); R2323 or 13-ethyl-17a-ethynl- 17B-hydroxygona-4,9,11-trien-3-one, Unlisted Drugs, 23(3): 34(b) (1971);MER-25; U-11,555A; U-11,100A; ICI-46,669 and ICI-46,474; all shown in L. Terenius, et al., "Aspects on the Mode of Action of Antiestrogens and Antiprogestrogens," Hormones and Antagonists. Gynec. Invest. 3: 98; Diphenol hydrochrysene; erythro-MEA; and Park Davis CN-55,945; all disclosed in C. Geynet, et al., "Estrogens and Antiestrogens," Hormones and Antagonists. Gynec. Invest 3: 12-13 (1972); Allenolic acid and cyclofenyl, disclosed in C. Geynet, et aL, Hormones and Antagonists. Gynec. Invest. 3:17 (1972); Chlorotrianisene, Merck Index, 10th ed., #2149; Ethamoxytriphetol, Merck Index, 10th ed., #3668; and Triparanol, Merck Index, 10th ed., #9541 and U.S. Pat. No. 2,914,562.

Various embodiments of dosage ranges are contemplated by this invention. In one embodiment, the dosage is in the range of 1-80 mg/day. In another embodiment, the dosage is in the range of 5-80 mg/day. In another embodiment the dosage is in the range of 35-66 mg/day. In another embodiment the dosage is in the range of 20-80 mg/day. In another embodiment the dosage is in the range of 20-60 mg/day. In another embodiment the dosage is in the range of 40-60 mg/day. In another embodiment the dosage is in a range of 45-60 mg/day. In another embodiment the dosage is in the range of 15-25 mg/day. In another embodiment the dosage is in the range of 55-65 mg/day. In one embodiment, the dosage is 20 mg/day. In another embodiment, the dosage is 40 mg/day. In another embodiment, the dosage is 60 mg/day. In another embodiment, the dosage is 80 mg/day.

In another embodiment the dosage is in the range of 50-80 mg/day. In another embodiment the dosage is in the range of 55-75 mg/day. In another embodiment the dosage is in the range of 60-70 mg/day. In another embodiment the dosage is in the range of 62-68 mg/day. In another embodiment the dosage is in the range of 58-62 mg/day. In another embodiment the dosage is in the range of 52-68 mg/day. Each dosage range represents a separate embodiment of the present invention.

Accordingly, the present invention provides, in one embodiment, a method of treating androgen-deprivation induced osteoporosis in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of preventing androgen-deprivation induced osteoporosis in a male subject having prostate cancer, the method comprising the step of administering to said subject an ami-estrogen agent and/or its analog, derivative, isomer, metabolite, pharmaceutically acceptable salt, pharmaceutical product, hydrate, N-oxide, or any combination thereof.

In another embodiment, the present invention provides a method of suppressing or inhibiting androgen-deprivation induced osteoporosis in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of reducing the incidence of androgen-deprivation induced osteoporosis in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of treating androgen-deprivation induced loss of BMD in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of preventing androgen-deprivation induced loss of BMD in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of suppressing or inhibiting androgen-deprivation induced loss of BMD in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of reducing the incidence of androgen-deprivation induced loss of BMD in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of treating androgen-deprivation induced bone fractures in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of preventing androgen-deprivation induced bone fractures in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of suppressing or inhibiting androgen-deprivation induced bone fractures in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of reducing the incidence of androgen-deprivation induced bone fractures in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

As provided herein, the results demonstrate that administration of an anti-estrogen, such as, for example, toremifene, is bone sparing. This was determined by measuring the levels of bone-specific serum markers that indicate bone resorption and formation. Further, this invention demonstrates that an anti-estrogen, such as, for example, toremifene (and/or 17-β -Estradiol), increases bone mineral density.

In another embodiment, the present invention provides a method of treating hot flashes in a subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of suppressing or inhibiting hot flashes in a subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of reducing the incidence of hot flashes in a subject, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of treating hot flashes in subject, the method comprising the step of administering to said subject toremifene and/or its analog, derivative, isomer, metabolite, pharmaceutically acceptable salt, pharmaceutical product, hydrate, N-oxide, or any combination thereof.

In another embodiment, the present invention provides a method of suppressing or inhibiting hot flashes in a subject, the method comprising the step of administering to said subject toremifene and/or its analog, derivative, isomer, metabolite, pharmaceutically acceptable salt, pharmaceutical product, hydrate, N-oxide, or any combination thereof.

In another embodiment, the present invention provides a method of reducing the incidence of hot flashes in a subject, the method comprising the step of administering to said subject toremifene and/or its analog, derivative, isomer, metabolite, pharmaceutically acceptable salt, pharmaceutical product, hydrate, N-oxide, or any combination thereof.

In one embodiment, the term "hot flash" refers to an episodic disturbance in body temperature, e.g., a sudden elevation in body temperature in a subject. In another embodiment, the disturbance is accompanied by perspiration. In one embodiment, the term "hot flashes" refers to a sudden feeling of heat in the upper part or all ofthe body, face and neck flush, red blotches appearing on the chest, back and arms, heavy sweating, cold shivering, etc, or any combination thereof In one embodiment, the hot flash is experienced by a human subject, in another embodiment, a male subject. In one embodiment, the hot flash is a result of ADT. In another embodiment, the hot flash is not a result of ADT.

The methods of the invention for treating hot flashes can be used, in one embodiment, to treat hot flashes that result from, for example, menopause, tamoxifen acetate treatment, prostate cancer treatment, alcohol dehydrogenase deficiency, or carcinoid syndrome/pheochromocytoma. Each type of hot flash represents a separate embodiment of the present invention.

As provided herein, the results demonstrate that administration of an anti-estrogen, such as, for example, toremifene, decreased the frequency of hot flashes and lowered serum FSH levels.

In another embodiment, the present invention provides a method of treating gynecomastia in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of suppressing or inhibiting gynecomastia in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of reducing the incidence of gynecomastia in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of treating gynecomastia in a male subject, the method comprising the step of administering to said subject toremifene and/or its analog, derivative, isomer, metabolite, pharmaceutically acceptable salt, pharmaceutical product, hydrate, N-oxide, or any combination thereof.

In another embodiment, the present invention provides a method of suppressing or inhibiting gynecomastia in a male subject, the method comprising the step of administering to said subject toremifene and/or its analog, derivative, isomer, metabolite, pharmaceutically acceptable salt pharmaceutical product, hydrate, N-oxide, or any combination thereof.

In another embodiment, the present invention provides a method of reducing the incidence of gynecomastia in a male subject, the method comprising the step of administering to said subject toremifene and/or its analog, derivative, isomer, metabolite, pharmaceutically acceptable salt, pharmaceutical product, hydrate, N-oxide, or any combination thereof.

In one embodiment, gynecomastia is characterized by an increased amount of breast tissue in males. In another embodiment, gynecomastia is mediated by estrogen. In another embodiment gynecomastia results from disturbances of the normal ratio of active androgen to estrogen in plasma or within the breast itself In another embodiment, gynecomastia results from estradiol formation. In one embodiment, the estradiol formation results from conversion of the circulating androgens to estrogens in peripheral tissues. In another embodiment gynecomastia is characterized by breast pain. In one embodiment gynecomastia is characterized by increased breast size, increased breast swelling, an increase in nipple to anterior axillary line, nipple to sternal notch, or a combination thereof.

In another embodiment, the present invention provides a method of reducing the incidence of hair loss in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of treating hair loss in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In another embodiment, the present invention provides a method of suppressing or inhibiting hair loss in a male subject having prostate cancer, the method comprising the step of administering to said subject an anti-estrogen agent and/or its analog, derivative, isomer, metabolite, pharmaceutically acceptable salt, pharmaceutical product, hydrate, N-oxide, or any combination thereof.

In one embodiment, "hair loss" refers to alopecia. In another embodiment, hair loss is due to a disruption in hair renewal which occasions, in a first stage, an acceleration of the frequency of the cycles, at the expense of the quality of the hair and then at the expense of its quantity. A gradual depletion of the head of hair takes place, in one embodiment, by regression of the so-called "terminal" hairs at the downy stage. Certain regions are, in one embodiment, preferentially affected, in particular the temples or frontal bulbs in men; while in women, diffuse alopecia of the vertex is observed. The term "alopecia" refers, in another embodiment, to the entire family of afflictions of the hair follicle, the final consequence of which is the partial or general permanent loss of the hair. In one embodiment, the hair loss is a result of ADT. In another embodiment, the hair loss is not a result of ADT. Each type of hair loss represents a separate embodiment of the present invention.

In one embodiment the anti-estrogen is a selective estrogen receptor modulator (SERM). In another embodiment, the anti-estrogen is a triphenylethylene. In another embodiment, the anti-estrogen is toremifene. In another embodiment, the anti-estrogen is toremifene citrate.

Osteoporosis is, in one embodiment, a systemic skeletal disease, characterized by low bone mass and deterioration of bone tissue, with a consequent increase in bone fragility and susceptibility to fracture. In osteoporotic patients, bone strength is abnormal, with a resulting increase in the incidence of fracture. Osteoporosis depletes both the calcium and the protein collagen normally found in the bone, resulting in either abnormal bone quality or decreased bone density. Bones that are affected by osteoporosis can fracture with only a minor fall or injury that normally would not cause a bone fracture. The fracture can be, in one embodiment, either in the form of cracking (as in a hip fracture) or collapsing (as in a compression fracture of the spine). The spine, hips, and wrists are common areas of osteoporosis bone fractures, although fractures can also occur in other skeletal areas.

In one embodiment, the osteoporosis is a result from ADT. In another embodiment, the osteoporosis is not a result of ADT. Each type of osteoporosis represents a separate embodiment of the present invention.

BMD is a measured calculation of the true mass of bone. The absolute amount of bone as measured by bone mineral density (BMD) generally correlates with bone strength and its ability to bear weight By measuring BMD, it is possible to predict fracture incidence in the same manner that measuring blood pressure can help predict the incidence of stroke.

BMD in one embodiment can be measured by known bone-mineral content mapping techniques. Bone density of the hip, spine, wrist, or calcaneus may be measured by a variety of techniques. The preferred method of BMD measurement is dual-energy x-ray densitometry (DXA). BMD of the hip, antero-posterior (AP) spine, lateral spine, and wrist can be measured using this technology. Measurement at any site predicts overall incidence of fracture, but information from a specific site is the best predictor of fracture at that site. Quantitative computerized tomography (QCT) is also used to measure BMD of the spine., See for example, "Nuclear Medicine: "Quantitative Procedures"by Wahner H W, Dunn W L, Thorsen H C, et al, published by Toronto Little, Brown & Co., 1983, (see pages 107-132). An article entitled "Assessment of Bone Mineral Part 1" appeared in the Journal of Nuclear Medicine, pp 1134-1141, (1984). Another article entitled "Bone Mineral Density of The Radius" appeared in Vol. 26, No. 11, (1985) Nov. Journal of Nuclear Medicine at pp 13-39. Abstracts on the use of gamma cameras for bone-mineral content measurements are (a) S. Hoory et al, Radiology, Vol. 157(P), p. 87 (1985), and (b) C. R. Wilson et al, Radiology, Vol. 157(P), p. 88 (1985).

The present invention provides, in one embodiment, a safe and effective method for treating, preventing; suppressing, inhibiting or reducing the incidence of androgen-deprivation induced osteoporosis and/or loss of BMD and is particularly useful for treating male subjects having prostate cancer having an elevated incidence of developing androgen-deprivation induced osteoporosis. In one embodiment, the male subject is a mammalian subject. In another embodiment, the male subject is a human subject. Each possibility represents a separate embodiment of the present invention.

In one embodiment, the subject has prostate cancer. In another embodiment, the subject has benign prostate hyperplasia. In another embodiment, the subject has a lower-than-normal or higher-man-normal level of an androgen, a testosterone, or an estrogen. In another embodiment, the subject has a hormone imbalance. Each possibility represents a separate embodiment of the present invention.

In another embodiment, the subject has received ADT. The terms "has received," "have received," and the like refer, in one embodiment, to subjects that have recently (within the last 6 months) or are currently receiving any treatment or therapy known in the art that reduces androgen levels in general or testosterone levels in particular. In another embodiment, the terms refer to a subject that received such a treatment or therapy more than 6 months previously. In one embodiment, the treatment or therapy is surgical. In another embodiment, the treatment or therapy is medical. In another embodiment, the treatment or therapy eliminates an androgen or a testosterone entirely, or below detectable levels. In another embodiment, the ADT is a side effect of a treatment or therapy not intended to reduce androgen or testosterone levels. Each of these possibilities represents a separate embodiment of the present invention.

The data presented herein demonstrate that anti-estrogens affect manifestations of androgen deprivation such as osteoporosis, bone loss, loss of BMD, hot flashes, gynecomastia, and hair loss. In one embodiment, the anti-estrogen of the present invention functions as an estrogen-receptor antagonist. In another embodiment, the anti-estrogen of the present invention functions as an estrogen-receptor agonist. In another embodiment, the anti-estrogen of the present invention functions as an estrogen-receptor antagonist in some tissues, and as an estrogen-receptor agonist in other tissues. Each possibility represents a separate embodiment of the present invention.

Furthermore, the anti-estrogens presented herein are effective at treating, suppressing or inhibiting osteopenia accompanied by bone loss. "Osteopenia" refers to decreased calcification or density of bone. This is a term that encompasses, in one embodiment, all skeletal systems in which such a condition is noted.

In other embodiments, the present invention provides a method of treating any disease, disorder, or symptom associated with ADT. In other embodiments, the present invention provides a method of treating any disease, disorder, or symptom associated with androgen deprivation. In other embodiments, the present invention provides a method of treating any disease, disorder, or symptom associated with testosterone deprivation. Each disease, disorder, or symptom represents a separate embodiment of the present invention.

As contemplated herein, the present invention relates to the use of an anti-estrogen compound and/or its analog, derivative, isomer, metabolite, pharmaceutically acceptable salt, pharmaceutical product, hydrate, N-oxide, or combinations thereof for treating, preventing, suppressing, inhibiting or reducing the incidence of androgen-deprivation induced osteoporosis, loss of BMD, hot flashes, gynecomastia, and/or hair loss. Thus, in one embodiment, the methods of the present invention comprise administering an analog of the anti-estrogen. In another embodiment, the methods of the present invention comprise administering a derivative of the anti-estrogen. In another embodiment, the methods of the present invention comprise administering an isomer of the anti-estrogen. In another embodiment, the methods of the present invention comprise administering a metabolite of the anti-estrogen. In another embodiment, the methods ofthe present invention comprise administering a pharmaceutically acceptable salt of the anti-estrogen. In another embodiment, the methods of the present invention comprise administering a pharmaceutical product of the anti-estrogen. In another embodiment, the methods of the present invention comprise administering a hydrate of the anti-estrogen. In another embodiment, the methods of the present invention comprise administering an N-oxide of the anti-estrogen. In another embodiment, the methods of the present invention comprise administering any of a combination of an analog, derivative, isomer, metabolite, pharmaceutically acceptable salt, pharmaceutical product, hydrate or N-oxide of the anti-estrogen.

As defined herein, the term "isomer" includes, but is not limited to, optical isomers and analogs, structural isomers and analogs, conformational isomers and analogs, and the like.

In one embodiment, this invention encompasses the use of various optical isomers of the anti-estrogen compound. It will be appreciated by those skilled in the art that the anti-estrogens of the present invention contain at least one chiral center. Accordingly, the anti-estrogens used in the methods of the present invention may exist in, and be isolated in, optically active or racemic forms. Some compounds may also exhibit polymorphism. It is to be understood that the present invention encompasses any racemic, optically active, polymorphic, or stereroisomeric form, or mixtures thereof, which form possesses properties useful in the treatment of androgen-related conditions described herein. In one embodiment, the anti-estrogens are the pure (R)-isomers. In another embodiment, the anti-estrogens are the pure (S)-isomers. In another embodiment, the anti-estrogens are a mixture of the (R) and the (S) isomers. In another embodiment, the anti-estrogens are a racemic mixture comprising an equal amount of the (R) and the (S) isomers. It is well known in the art how to prepare optically active forms (for example, by resolution of the racemic form by recrystallization techniques, by synthesis from optically active starting materials, by chiral synthesis, or by chromatographic separation using a chiral stationary phase).

The invention includes "pharmaceutically acceptable salts" of amino-substituted compounds with organic and inorganic acids, for example, citric acid and hydrochloric acid. The invention also includes N-oxides of the amino substituents of the compounds described herein. Pharmaceutically acceptable salts can also be prepared from the phenolic compounds by treatment with inorganic bases, for example, sodium hydroxide. Also, esters of the phenolic compounds can be made with aliphatic and aromatic carboxylic acids, for example, acetic acid and benzoic acid esters.

This invention further includes, in another embodiment derivatives of the anti-estrogens. The term "derivatives" includes but is not limited to ether derivatives, acid derivatives, amide derivatives, ester derivatives and the like. In addition, this invention further includes hydrates ofthe anti-estrogen compounds. The term "hydrate" includes but is not limited to hemihydrate, monohydrate, dihydrate, trihydrate and the like.

This invention further includes metabolites of the anti-estrogen compounds. The term "metabolite" means any substance produced from another substance by metabolism or a metabolic process.

This invention further includes pharmaceutical products of the anti-estrogen compounds. The term "pharmaceutical product" means a composition suitable for pharmaceutical use (pharmaceutical composition), as defined herein.

In addition, the invention encompasses pure (Z)- and (E)- isomers of the anti-estrogen compounds defined herein and mixtures thereof as well as pure (RR, SS)- and (RS, SR)-enantiomer couples and mixtures thereof.

### Pharmaceutical Compositions

In one embodiment, the methods of the present invention comprise administering a pharmaceutical composition comprising the anti-estrogen and/or its analog, derivative, isomer, metabolite, pharmaceutically acceptable salt, pharmaceutical product, hydrate, N-oxide, or any combination thereof; and a pharmaceutically acceptable carrier. The pharmaceutical composition is administered to a male subject having prostate cancer; for treating and/or preventing androgen-deprivation induced osteoporosis and/or loss of BMD; for suppressing or inhibiting androgen-deprivation induced osteoporosis and/or loss of BMD; and/or for reducing the incidence of androgen-deprivation induced osteoporosis and/or loss of BMD in the male subject.

As used herein, "pharmaceutical composition" means a "therapeutically effective amount" of the active ingredient, i.e. the anti-estrogen, together with a pharmaceutically acceptable carrier or diluent. A "therapeutically effective amount" as used herein refers to that amount which provides a therapeutic effect for a given condition and administration regimen.

The pharmaceutical compositions containing the anti-estrogen can be administered to a subject by any method known to a person skilled in the art, such as parenterally, paracancerally, transmucosally, transdermally, intramuscularly, intravenously, intradermally, subcutaneously, intraperitonealy, intraventricularly, intracranially, intravaginally or intratumorally.

In one embodiment, the pharmaceutical compositions are administered orally, and are thus formulated in a form suitable for oral administration, i.e. as a solid or a liquid preparation. Suitable solid oral formulations include tablets, capsules, pills, granules, pellets and the like. Suitable liquid oral formulations include solutions, suspensions, dispersions, emulsions, oils and the like. In one embodiment of the present invention, the anti-estrogen compounds are formulated in a capsule. In accordance with this embodiment, the compositions of the present invention comprise, in addition to the anti-estrogen active compound and the inert carrier or diluent, a hard gelating capsule.

Further, in another embodiment, the pharmaceutical compositions are administered by intravenous, intraarterial or intramuscular injection of a liquid preparation. Suitable liquid formulations include solutions, suspensions, dispersions, emulsions, oils and the like, In one embodiment, the pharmaceutical compositions are administered intravenously and are thus formulated in a form suitable for intravenous administration. In another embodiment, the pharmaceutical compositions are administered intraarterially and are thus formulated in a form suitable for intraarterial administration: In another embodiment, the pharmaceutical compositions are administered intramuscularly and are thus formulated in a form suitable for intramuscular administration.

Further, in another embodiment, the pharmaceutical compositions are administered topically to body surfaces and are thus formulated in a form suitable for topical administration. Suitable topical formulations include gels, ointments, creams, lotions, drops and the like. For topi cal administration, the anti-estrogen agents or their physiologically tolerated derivatives such as salts, esters, N-oxides, and the like are prepared and applied as solutions, suspensions, or emulsions in a physiologically acceptable diluent with or without a pharmaceutical carrier.

Further, in another embodiment the pharmaceutical compositions are administered as a suppository, for example a rectal suppository or a urethral suppository. Further, in another embodiment, the pharmaceutical compositions are administered by subcutaneous implantation of a pellet. In a further embodiment, the pellet provides for controlled release of anti-estrogen agent over a period of time.

In another embodiment, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

As used herein "pharmaceutically acceptable carriers or diluents" are well known to those skilled in the art. The carrier or diluent may be a solid carrier or diluent for solid formulations, a liquid carrier or diluent for liquid formulations, or mixtures thereof.

Solid carriers/diluents include, but are not limited to, a gum, a starch (e.g. corn starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

For liquid formulations, pharmaceutically acceptable carriers may be aqueous or non-aqueous solutions, suspensions, emulsions or oils. Examples of non-aqueous solvents are propylene glycol, polyethylene glycol, and injectable organic esters such as ethyl oleate. Aqueous carriers include water, alcoholic/aqueous solutions, emulsions or suspensions, including saline and buffered media. Examples of oils are those of petroleum, animal, vegetable, or synthetic origin, for example, peanut oil, soybean oil, mineral oil, olive oil, sunflower oil, and fish-liver oil.

Parenteral vehicles (for subcutaneous, intravenous, intraarterial, or intramuscular injection) include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Examples are sterile liquids such as water and oils, with or without the addition of a surfactant and other pharmaceutically acceptable adjuvants. In general, water, saline, aqueous dextrose and related sugar solutions, and glycols such as propylene glycols or polyethylene glycol are preferred liquid carriers, particularly for injectable solutions. Examples of oils are those of petroleum, animal, vegetable, or synthetic origin, for example, peanut oil, soybean oil, mineral oil, olive oil, sunflower oil, and fish-liver oil.

In addition, the compositions may further comprise binders (e.g. acacia, cornstarch, gelatin, carbomer, ethyl cellulose, guar gum, hydroxypropyl cellulose, hydroxy propyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents(e.g. carbomer, colloidal silicon dioxide, ethyl cellulose, guar gum), sweeteners (e.g. aspartame, citric acid), preservatives (e,g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

In one embodiment, the pharmaceutical compositions provided herein are controlled-release compositions, i.e. compositions in which the anti-estrogen compound is released over a period of time after administration. Controlled- or sustained-release compositions include formulation in lipophilic depots (e.g. fatty acids, waxes, oils). In another embodiment, the composition is an immediate-release composition, i.e. a composition in which all of the anti-estrogen compound is released immediately after administration.

In yet another embodiment, the pharmaceutical composition can be delivered in a controlled release system. For example, the agent may be administered using intravenous infusion, an implantable osmotic pump, a transdermal patch, liposomes, or other modes of administration. In one embodiment, a pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1959). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled-release systems are discussed in the review by Langer (Science 249:1527-1533 (1990).

The compositions may also include incorporation ofthe active material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions will influence the physical state, solubility, stability, rate of *in vivo* release, and rate of *in* vivo clearance.

Also comprehended by the invention are particulate compositions coated with polymers (e.g. poloxamers or poloxamines) and the compound coupled to antibodies directed against tissue-specific receptors, ligands or antigens or coupled to ligands of tissue-specific receptors,

Also comprehended by the invention are compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. The modified compounds are known to exhibit substantially longer half-lives in blood following intravenous injection than do the corresponding unmodified compounds (Abuchowski et al., 1981; Newmark et al., 1982; and Katre et al., 1987). Such modifications may also increase the compound's solubility in aqueous solution, eliminate aggregation, enhance the physical and chemical stability of the compound, and greatly reduce the immunogenicity and reactivity of the compound. As a result, the desired *in vivo* biological activity may be achieved by the administration of such polymer-compound abducts less frequently or in lower doses than with the unmodified compound.

The preparation of pharmaceutical compositions that contain an active component, for example by mixing, granulating, or tablet-forming processes, is well understood in the art. The active therapeutic ingredient is often mixed with excipients that are pharmaceutically acceptable and compatible with the active ingredient. For oral administration, the anti-estrogen agents or their physiologically tolerated derivatives such as salts, esters, N-oxides, and the like are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions. For parenteral administration, the anti-estrogen agents or their physiologically tolerated derivatives such as salts, esters, N-oxides, and the like are converted into a solution, suspension, or emulsion, if desired with the substances customary and suitable for this purpose, for example, solubilizers or other substances.

An active component can be formulated into the composition as neutralized pharmaceutically acceptable salt forms. Pharmaceutically acceptable salts include the acid addition salts (formed with the free amino groups of the polypeptide or antibody molecule), which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

For use in medicine, the salts of the anti-estrogens are pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts, which may, for example, be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic: acid, oxalic acid, citric acid, tartaric acid, carbonic acid or phosphoric acid.

The term "contacting" means, in one embodiment, that the anti-estrogen compound of the present invention is introduced into a sample containing the enzyme in a test tube, flask, tissue culture, chip, array, plate, microplate, capillary, or the like, and incubated at a temperature and time sufficient to permit binding of the anti-estrogen to the enzyme. Methods for contacting the samples with the anti-estrogen or other specific binding components are known to those skilled in the art and may be selected depending on the type of assay protocol to be run. Incubation methods are also standard and are known to those skilled in the art.

In another embodiment, the term "contacting" means that the anti-estrogen compound of the present invention is introduced into a subject receiving treatment, and the anti-estrogen compound is allowed to come in contact with the androgen receptor *in vivo.*

As used herein, the term "treating" includes preventative as well as disorder remitative treatment. As used herein, the terms "reducing", "suppressing" and "inhibiting" have their commonly understood meaning of lessening or decreasing. As used herein, the term "progression" means increasing in scope or severity, advancing, growing or becoming worse. As used herein, the term "recurrence" means the return of a disease after a remission.

As used herein, the term "administering" refers to bringing a subject in contact with an anti-estrogen compound of the present invention. As used herein, administration can be accomplished *in vitro,* i.e. in a test tube, or *in vivo,* i.e. in cells or tissues of living organisms, for example humans. In one embodiment, the present invention encompasses administering the compounds of the present invention to a subject.

In one embodiment, the methods of the present invention comprise administering an anti-estrogen compound as the sole active ingredient However, also encompassed within the scope of the present invention are methods for hormone therapy, for treating prostate cancer, for delaying the progression of prostate cancer, and for preventing and/or treating the recurrence of prostate cancer, which comprise administering the anti-estrogen compounds in combination with one or more therapeutic agents. These agents include, but are not limited to: LHRH analogs, reversible anti-androgens (such as bicalutamide or flutamide), additional anti-estrogens, anticancer drugs, 5-alpha reductase inhibitors, aromatase inhibitors, progestins, selective androgen receptor modulators (SARMS) or agents acting through other nuclear hormone receptors.

Thus, in one embodiment, the methods of the present invention include using compositions and pharmaceutical compositions comprising an anti-estrogen, in combination with an LHRH analog. In another embodiment, the methods ofthe present invention include using compositions and pharmaceutical compositions comprising an anti-estrogen, in combination with a reversible anti-androgen. In another embodiment, the methods of the present invention include using compositions and pharmaceutical compositions comprising an anti-estrogen, in combination with an additional anti-estrogen. In another embodiment, the methods of the present invention include using compositions and pharmaceutical compositions comprising an anti-estrogen, in combination with an anticancer drug. In another embodiment, the methods of the present invention include using compositions and pharmaceutical compositions comprising an anti-estrogen, in combination with a 5-alpha reductase inhibitor. In another embodiment, the methods ofthe present invention include using compositions and pharmaceutical compositions comprising an anti-estrogen, in combination with an aromatase inhibitor. In another embodiment, the methods of the present invention include using compositions and pharmaceutical compositions comprising an anti-estrogen, in combination with a progestin. In another embodiment, the methods ofthe present invention include using compositions and pharmaceutical compositions comprising an anti-estrogen, in combination with a SARM. In another embodiment, the methods of the present invention include using compositions and pharmaceutical compositions comprising an anti-estrogen, in combination with an agent acting through other nuclear hormone receptors.

The following examples are presented in order to more fully illustrate the preferred embodiments ofthe invention. They should in noway, however, be construed as limiting the broad scope of the invention.
**Embodiments of the invention are summarized in the following items:**
- **1.**: Use of toremifene or its analog, derivative, isomer, metabolite, pharmaceutically acceptable salt, pharmaceutical product, hydrate, N-oxide, or any combination thereof in the preparation of a composition for use in treating, reducing the incidence of, suppressing, or inhibiting hot flashes in a subject.
- **2.**: The use according to item 1, wherein said toremifene is toremifene citrate.
- **3.**: The use according to item 1, wherein said composition comprises a pharmaceutically acceptable carrier.
- **4.**: The use according to item 1, wherein said composition is prepared in liquid form for intravenous, intraarterial, or intramuscular injection; in pellet form for subcutaneous implantation; in a liquid or solid form for oral administration; or for topical application.
- **5.**: The use according to item 4, wherein said composition is a pellet, a tablet, a capsule, a solution, a suspension, an emulsion, an elixir, a gel, a cream, a suppository or a parenteral formulation.
- **6.**: The use according to item 1, wherein sais subject is human.
- **7.**: The use according to item 6, wherein said human subject is male.
- **8.**: The use according to item 7, wherein said male subject has received androgen deprivation therapy (ADT).
- **9.**: Use of toremifene and/or its analog, derivative, isomer, metabolite, pharmaceutically acceptable salt, pharmaceutical product, hydrate, N-oxide, or any combination thereof in the preparation of a composition for use in treating, reducing the incidence of, suppressing, or inhibiting gynecomastia in a subject.
- **10.**: The use according to item 9, wherein said toremifene is toremifene citrate.
- **11.**: The use according to item 9, wherein said composition comprises a pharmaceutically acceptable carrier.
- **12.**: The use according to item 9, wherein said pharmaceutical composition is prepared in liquid form for intravenous, intraarterial, or intramuscular injection; in pellet form for subcutaneous implantation; in a liquid or solid form for oral administration; or for topical application.
- **13.**: The use according to item 12, wherein said pharmaceutical composition is a pellet, a tablet, a capsule, a solution, a suspension, an emulsion, an elixir, a gel, a cream, a suppository or a parenteral formulation.
- **14.**: The use according to item 9, wherein said subject is human.
- **15.**: The use according to item 14, wherein said human subject is male.
- **16.**: The use according to item 16, wherein said male subject has received androgen deprivation therapy (ADT).

### EXPERIMENTAL DETAILS SECTION

### EXAMPLE 1

### EFFECT OF TOREMIFENE ON OSTEOPENIA IN MALE RATS

### Introduction and Study Aims

The purpose of these studies is: 1) To determine the efficacy of two doses of toremifene (Tor) in the prevention of bone loss in orchidsctomized rats (ORX) as determined by bone mineral density analyses with dual energy X-ray absorptiometty (DXA) and ash mineral density; 2) To determine the efficacy of two doses of toremifene on the mechanical strength of bone compared to vehicle-treated ORX and sham-operated rats; and to evaluate the effects of two doses of toremifene on histomorphometric indices of bone turnover and bone structure in ORX rats.

### Experimental Design

Eight month-old male Sprague Dawley rats were either sham-operated (SHAM) or orchidectomized (ORX). Treatment began immediately following surgery. The treatment groups were as described in Table 1.

**Table 1: Treatment Groups**

| | **Description** | **Sacrifice** |
|---|---|---|
| 1 | Sham + vehicle | 10 animals at wk 6 |
| 2 | Sham + vehicle | 10 animals at wk 12 |
| 3 | ORX + vehicle | 10 animals at wk 6 |
| 4 | ORX + vehicle | 10 animals at wk 12 |
| 5 | ORX + 5 mg/kg/d toremifene | 10 animals at wk 6 |
| 6 | ORX + 5 mg/kg/d toremifene | 10 animals at wk 12 |
| 7 | ORX + 10 mg/kg/d toremifene | 10 animals at wk 6 |
| 8 | ORX +10 mg/kg/d toremifene | 10 animals at wk 12 |
| 9 | ORX + 0.2 mg/kg/d 17β-estradiol | 10 animals at wk 6 |
| 10 | ORX + 0.2 mg/kg/d 17β-estradiol | 10 animals at wk 12 |

Animals were given calcein (intraperitoneally) 12 and 2 days prior to sacrifice. Femurs and lumbar vertebra (L5) were preserved frozen wrapped in saline-soaked gauze. Tibiae and lumbar vertebrae (L4) were preserved in 10% formalin for 48 hours, then transferred to 70% ethanol for long-term storage.

### Bone Analysis Techniques

Bone sites were subjected to different analysis techniques at each time point harvested, as shown in Table 2.

**Table 2: Analysis Techniques**

| **Technique /Bone Site** | **Week 6 Specimens** | **Week 12 Specimens** |
|---|---|---|
| DXA of the right femur | Yes | Yes |
| DXA of the lumbar vertebra (L5-L6) | No | Yes |
| Histology and histomorphometry of the right proximal tibia | Yes | Yes |
| Histology and histomorphometry of the lumbar vertebra (L4) | No | Yes |
| Ash density and Ca and Pi content in ash of the right femur | No | Yes |
| Biomechanical testing of the distal segment of the left femur | Yes | Yes |
| Biomechanical testing of the femoral shaft of the left femur | No | Yes |
| Biomechanical testing of the lumbar vertebral body (L5) | No | Yes |
| Biomechanical testing of the femoral neck of the left femur | No | Yes |

### Ex Vivo Dual Energy X-Ray Absorptiometry (DXA)

*Ex vivo* DXA analysis was performed on the vertebra (L5) using a PIXImus instrument and associated animal research software (Lunar Corporation, Madison, WI). Bone mineral content (BMC), bone area (BA) and bone mineral density (BMD) are reported. *Ex vivo* DXA analysis was performed on the femur using a pDXA Sabre and associated animal research software (Norland Medical Systems, Inc., Fort Atkinson, WI). BMC, BA and BMD are reported for four regions of interest: whole femur, 25% distal femur, 25% proximal femur and 50% of midshaft femur.

**Histology and Static and Dynamic Bone Histomorphometry:** Approximately 1.1 cm of the proximal tibia was cut away and the frontal face was trimmed with a low-speed diamond wheel saw to expose the marrow cavity. The L4 lumbar vertebral body was prepared from the lumbar-vertebra by trimming off the processes with a slow-speed diamond saw. Each proximal tibia and lumbar vertebral body was dehydrated in a series of ascending ethanol (EtOH) concentrations. Following dehydration, the bone samples were infiltrated and then embedded in a methyl methacrylate-dibutyl phthalate plastic composite. Embedded samples of the proximal tibiae were sectioned using a Leitz motorized rotary microtome equipped with a tungsten-carbide microtome knife. Once the blocks had been trimmed and faced to the sampling site, a 4-micron section was stained with Goldner's trichrome stain for bright field microscopy and an 8-micron section was left unstained for epifluorescent microscopy. Embedded lumbar vertebral body samples were also sectioned using a Leitz motorized rotary microtome equipped with a tungsten-carbide microtome knife. Once the blocks had been trimmed and faced to the sampling site, a 4-micron section was stained with Goldner's trichrome stain for bright field microscopy and an 8-micron section was left unstained for epifluorescent microscopy.

The cancellous bone in the secondary spongiosa of the proximal tibia was evaluated in a region of interest (ROI) that was 1.0 mm distal to the lowest point ofthe growth plate in a rectilinear region of 3 x 2 fields. The cancellous bone in the marrow cavity of the lumbar vertebral body was evaluated in a ROI that was approximately 0.5 mm away from the end plates and dorsal and ventral cortices.

The bone histomorphometry was performed using an OsteoMeasure software program (OsteoMetrics, Inc., Atlanta, GA) interfaced with a Nikon Eclipse E400 light/epifluorescent microscope and video subsystem. All slides were analyzed in a blinded manner. Total tissue area, trabecular bone area, trabecular bone perimeter and osteoclast perimeter and number were measured on 4 µm Goldner's trichrome-stained sections. Percent trabecular bone area, trabecular number, trabecular thickness, trabecular separation, osteoclast perimeter as a percentage of bone surface and osteoclast number per unit of bone surface were then calculated according to standardized formulae. For deriving the dynamic parameters, single-labeled calcein perimeter, double-labeled calcein perimeter and interlabel width (label thickness) were measured on 8 µm unstained sections. Mineralizing surface, mineral apposition rate and bone formation rate-surface, bone and tissue referents were calculated.

### Ash Density and Ca (calcium) and Pi Contents in Ash

Femur was cleaned of flesh and its volume determined using Archimedes principle. Wet weight of the femur in air and in water were separately collected and volume calculated. Dry weight was obtained after the femur was dried in the drying oven. The femur was then ashed in a muffle furnace at 600°C for at least 10 hours and the mineral content of the ashed femur was weighed. The ashed femur was then subjected to calcium and phosphorus content determinations using a routine chemistry instrument.

### Measurement of Bone Mechanical Properties

Bone strength was evaluated using a compression test at the vertebral body, a three point bending test at the femoral shaft, a compression test at the distal femur and a cantilever compression test of the femoral neck. Prior to mechanical testing, all samples were thawed in cold saline and carefully cleaned of any remaining adherent soft tissue.

### RESULTS

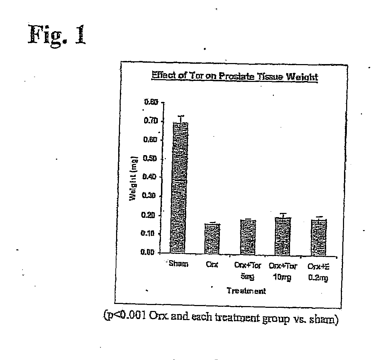

***Prostate weight*:** Orx decreased prostate weight by 77% but E (Estradiol) or Tor (toremifene) had no effect on prostate weight in the Orx rat (Figure 1).

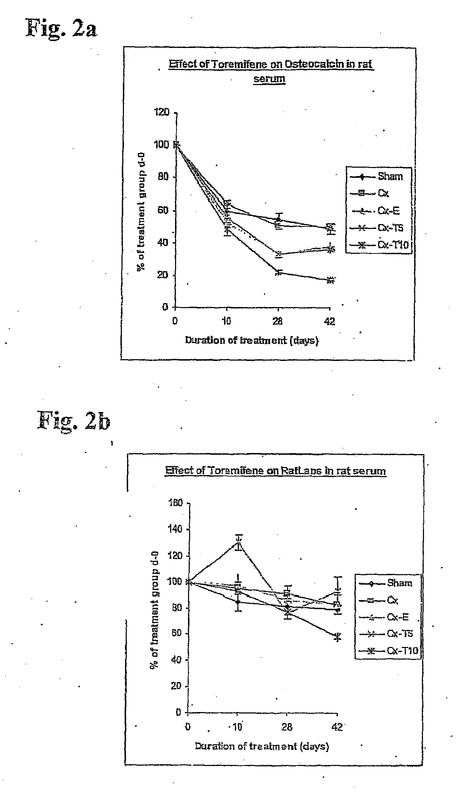

***Bone Biochemical Markers:*** OC levels in Orx rats were slighlty higher than the sham group at 10 days and treatment with E, 5 mg-Tor and 10 mg-Tor all reversed OC levels to slightly below that of Sham group. Further effect of Tor was dose- and time-dependent (Figures 2a and 2b). Thus, the inhibitory effect of 5mg-Tor on OC levels at 28 days was same as E and did not change further whereas that of 10mg-Tor was more pronounced than both 5mg Tor and E, and continued at 42 days.

CTX levels at all time points were slightly higher in Orx rats compared to the sham-operated rats. Neither E nor Tor had any effect on CTX at 10 days. However, continued treatment with E and 10 mg Tor reduced the CTX levels at 28 days, Tor being slightly more effective than E. While CTX levels did not change further with E, 10mg-Tor treated rats had significantly lower CTX levels at 42 days when the CTX levels declined to levels lower than sham or E-treated group. Thus, the anti-resorptive activity of 10 mg-Tor continued with time and was significantly higher than E at 42 days. The effect of 5 mg Tor on CTX was not uniform and cannot be interpreted.

***Static and Dynamic Bone Histomorphometry of Proximal Tibia:*** Orx induced a significantly elevated bone turnover state, as seen by increased osteoclast number, mineral apposition rate (MAR) and bone formation rate (BFR/BS, BFR/BV, BFR/TV) resulting in reduced trabecular bone volume at the proximal tibia. This reduction in trabecular bone volume was due to a reduction in trabecular bone thickness and number, although none of these changes reached statistically significant levels. Treatment with 10 mg/kg/d Tor and E was very effective in preventing loss of trabecular bone volume.

The Orx-induced increase in bone turnover parameters was significantly inhibited by Tor and E treatment (p <0.05 vs. Orx + vehicle). Tor 5 and 10 mg/kg/d significantly reduced the Orx-induced elevation in bone turnover parameters, with results similar to treatment with 0.2 mg/kg/d E. Osteoclast number and surface were reduced to the levels of sham-operated animals while bone formation rate parameters were reduced to levels below those of sham-operated animals by either Tor or E. The reduction in bone formation rate was the result of reduced bone forming surface (mineralizing surface) and bone forming activity (MAR) after Tor or E treatment. Thus, estradiol and toremifene treatment resulted in improved bone microarchitecture. Summarized data is shown in **Tables 3a and 3b.**

**Table 3a: Static and Cellular Parameters at the Proximal Tibia at 6 Wks**

| **Treatment Group** | | **Trabecular Bone Volume** | **Trabecular Thickness** | **Trabecular Number** | **Trabecular Separation** | **Osteoclast Surface** | **Osteoclast Number/ Bone Surface** |
|---|---|---|---|---|---|---|---|
| | | **%** | **?m** | **#/mm** | **? m** | **%** | **#/mm** |
| Sham | mean | 8.77 | 50.14 | 1.70 | 582 | 5.43 | 3.11 |
| vehicle | SD | 3.62 | 6.69 | 0.49 | 183 | 1.14 | 0.58 |
| | n | 8 | 8 | 8 | 8 | 8 | 8 |
| | stat | n.s. | n.s. | n.s. | n.s. | n.s. | ** |
| Orx | mean | 6.08 | 41.91 | 1.42 | 689.67 | 9.47 | 4.92 |
| vehicle | SD | 2.17 | 7.56 | 0.29 | 152.24 | 2.19 | 1.21 |
| | n | 9 | 9 | 9 | 9 | 9 | 9 |
| | stat | n.a. | n.a. | n.a. | n.a. | n.a. | n.a. |
| Orx | mean | 533 | 41.39 | 1.30 | 837.67 | 6.61 | 3.98 |
| 5 mg/kg/d | SD | 1.86 | 7.94 | 0.40 | 433.65 | 1.97 | 1.39 |
| Toremifene | n | 9 | 9 | 9 | 9 | 9 | 9 |
| | stat | n.s. | n.s. | n.s. | n.s. | n.s. | n.s. |
| Orx | mean | 8.81 | 45.54 | 1.85 | 570.89 | 5.27 | 3.33 |
| 10 mg/kg/d | SD | 4.33 | 8.81 | 0.66 | 256.87 | 1.40 | 0.82 |
| Toremifene | n | 9 | 9 | 9 | 9 | 9 | 9 |
| | stat | n.s. | n.s. | n.s. | n.s. | n.s. | ** |
| Orx | mean | 8.26 | 47.11 | 1.75 | 572 | 6.35 | 3.77 |
| 0.2 mg/kg/d | SD | 2.34 | 4.37 | 0.46 | 226 | 2.33 | 1.30 |
| Estradiol | n | 8 | 8 | 8 | 8 | 8 | 8 |
| | stat | n.s. | n.s. | n.s. | n.s. | n.s. | n.s. |

**Table 3b. Dynamic Parameters at the Proximal Tibia at 6 Wks**

| **Treatment Group** | | **Mineralizing Surface** | **Mineral Apposition Rate** | **Bone Form. Rate, Bone Surface Ref** | **Bone Form Rate, Bone Vol. Ref** | **Bone Formation Rate, Tissue Volume Ref** |
|---|---|---|---|---|---|---|
| | | **%** | **um/day** | **um³/um²/yr** | **%/yr** | **%/yr** |
| Sham | mean | 9.02 | 0.81 | 30.00 | 126.01 | 9.55 |
| vehicle | SD | 3.27 | 0.41 | 19.80 | 93.63 | 5.33 |
| | n | 8 | 8 | 8 | 8 | 8 |
| | stat | ** | n.s. | ** | ** | ** |
| Orx | mean | 16.22 | 1.11 | 65.56 | 311.41 | 19.83 |
| vehicle | SD | 4.44 | 0.67 | 42.32 | 195.79 | 15.18 |
| | n | 9 | 9 | 9 | 9 | 9 |
| | stat. | n.a | n.a. | n.a. | n.a. | n.a. |
| Orx | mean | 6.67 | 0.32 | 10.78 | 58.20 | 2.30 |
| 5 mg/kg/d | SD | 4.82 | 0.31 | 12.00 | 63.81 | 2.42 |
| Toremifene | n | 9 | 9 | 9 | 9 | 9 |
| | stat | n.s. | ** | ** | ** | ** |
| Orx | mean | 3.02 | 0.34 | 4.78 | 21.26 | 2.07 |
| 10mg/kg/d | SD | 1.93 | 0.33 | 5.04 | 22.22 | 2.54 |
| Toremifene | n | 9 | 9 | 9 | 9 | 9 |
| | stat | n.s. | ** | ** | ** | ** |
| Orx | mean | 5.31 | 0.43 | 10.63 | 42.70 | 3.13 |
| 0.2 mg/kg/d | SD | 3.00 | 0.36 | 10.69 | 43.79 | 3.12 |
| Estradiol | n | 8 | 8 | 8 | 8 | 8 |
| | stat | n.s. | ** | ** | ** | ** |

| | | | | | | |
|---|---|---|---|---|---|---|
| n.a. not applicable; n.s. not significant; ** p < 0.05 vs. ORX + vehicle | | | | | | |

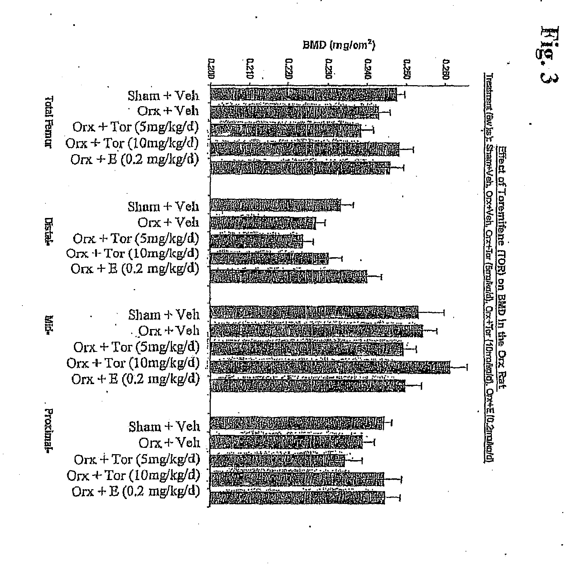

***Bone Mineral Density (BMD) of Femur*:** After 6 weeks oftreatment, excised femurs were subjected to DXA scan. The scan results were analyzed in four different regions of interest: whole femur, distal femur, midshaft femur and proximal femur. Summarized data is shown in Tables 4a and 4b, and Figure 3.

For the whole femur, Orx did not alter bone mass to any significant extent and treatment with toremifene or estradiol did not exhibit any significant effect either. The distal femur is a site that is rich in trabecular bone. At the distal femur, Orx increased bone area and as a result decreased bone density. Treatment with 10 mg/kg/d toremifene prevented Orx-related loss of bone density, the action of Tor being due to reduction in bone area. E, on the other hand, increased bone mineral content and thus significantly improved overall density (p<0.05 vs Orx+Vehicle). The midshaft femur is rich in cortical bone. Like the distal femur, the proximal femur is a trabecular bone rich site. At the proximal femur, Orx resulted in a small loss in bone mineral density. As in distal femur, while 5mg/kg/d Tor was ineffective, treatment with 10 mg/kg/d Tor and E was helpful in preventing this loss, the bone density with both treatments being at the level of sham control. Orx did not alter the bone mineral density at the midshaft femur and Tor or E also did not show any effect at this site.

**Table 4a: BMD of Whole Femur and Distal Femur at 6 wks**

| **Treatment Group** | **Data** | **Whole femur** | | | | **Distal femur** | | |
|---|---|---|---|---|---|---|---|---|
| | | **Bone Mineral Content** | | **Bone Area** | **Bone Mineral Density** | **Bone Mineral Content** | **Bone Area** | **Bone Mineral Density** |
| | | **gm** | | **cm²** | **gm/cm²** | **gm** | **cm²** | **gm/cm²** |
| Sham | mean | 0.588 | | 2.392 | 0.245 | 0.154 | 0.662 | 0.233 |
| vehicle | SD | 0.073 | | 0.253 | 0.008 | 0.012 | 0.057 | 0.009 |
| | n | 8 | | 8 | 8 | 8 | 8 | 8 |
| | stat | n.a. | | n.a. | n.a. | n.a | n.a. | n.a. |
| ORX | mean | 0.614 | | 2.525 | 0.243 | 0.154 | 0.678 | 0.227 |
| vehicle | SD | 0.028 | | 0.090 | 0.008 | 0.007 | 0.023 | 0.007 |
| | n | 8 | | 8 | 8 | 8 | 8 | 8 |
| | stat | n.s. | | n.s. | n.s. | n.s. | n.s. | n.s. |
| ORX | mean | 0.570 | | 2.389 | 0.238 | 0.145 | 0.649 | 0.224 |
| 5 mg/kg/d | SD | 0.043 | | 0.101 | 0.010 | 0.009 | 0.025 | 0.008 |
| Toremifene | n | 9 | 9 | | 9 | 9 | 9 | 9 |
| | stat | n.s. | n.s. | | n.s. | n.s. | n.s. | n.s. |
| ORX | mean | 0.601 | 2.420 | | 0.248 | 0.152 | 0.659 | 0.230 |
| 10 mg/kg/d | SD | 0.045 | 0.124 | | 0.011 | 0.012 | 0.036 | 0.010 |
| Toremifene | n | 9 | 9 | | 9 | 9 | 9 | 9 |
| | stat | n.s. | n.s. | | n.s. | n.s. | n.s. | n.s. |
| ORX | mean | 0.609 | 2.480 | | 0.246 | 0.162 | 0.674 | 0.240 |
| 0.2mg/kg/d | SD | 0.033 | 0.093 | | 0.010 | 0.009 | 0.022 | 0.010 |
| Estradiol | n | 8 | 8 | | 8 | 8 | 8 | 8 |
| | stat | n.s. | n.s. | | n.s. | n.s. | n.s. | ** |

| | | | | | | | | |
|---|---|---|---|---|---|---|---|---|
| n.a. not applicable; n.s. not significant; ** p < 0.05 vs. Orx + vehicle | | | | | | | | |

**Table 4b: BMD of Midshaft Femur and Proximal Femur at 6 wks**

| **Treatment Group** | **Data** | **Midshaft femur** | | | **Proximal femur** | | |
|---|---|---|---|---|---|---|---|
| | | **Bone Mineral Content** | **Bone Area** | **Bone Mineral Density** | **Bone Mineral Content** | **Bone Area** | **Bone Mineral Density** |
| | | **gm** | **cm²** | **gm/cm²** | **gm** | **cm²** | **gm/cm²** |
| Sham | mean | 0.283 | 1.114 | 0.253 | 0.151 | 0.618 | 0.244 |
| vehicle | SD | 0.037 | 0.087 | 0.019 | 0.031 | 0.128 | 0.005 |
| | n | 8 | 8 | 8 | 8 | 8 | 8 |
| | stat | n.a. | n.a. | n.a. | n.a. | n.a. | n.a. |
| ORX | mean | 0.295 | 1.160 | 0.254 | 0.166 | 0.697 | 0.239 |
| vehicle | SD | 0,016 | 0.054 | 0.010 | 0.008 | 0.030 | 0.009 |
| | n | 8 | 8 | 8 | 8 | 8 | 8 |
| | stat | n.s. | n.s. | n.s. | n.s. | n.s. | n.s. |
| ORX | mean | 0.271 | 1.086 | 0.249 | 0.154 | 0.658 | 0.234 |
| 5 mg/kg/d | SD | 0.023 | 0.055 | 0.011 | 0.014 | 0.038 | 0.013 |
| Toremifene | n | 9 | 9 | 9 | 9 | 9 | 9 |
| | stat | n.s. | n.s. | n.s. | n.s. | n.s. | n.s. |
| ORX | mean | 0.286 | 1.094 | 0.261 | 0.164 | 0.672 | 0.244 |
| 10 mg/kg/d | SD | 0.025 | 0.065 | 0.014 | 0.012 | 0.034 | 0.013 |
| Toremifene | n 9 | 9 | 9 | 9 | 9 | 9 | 9 |
| | stat | n.s. | n.s. | n.s. | n.s. | n.s. | n.s. |
| ORX | mean | 0.281 | 1.128 | 0.250 | 0.167 | 0.685 | 0.245 |
| 0.2 mg/kg/d | SD | 0.016 | 0.049 | 0.013 | 0.009 | 0.025 | 0.011 |
| Estradiol | n | 8 | 8 | 8 | 8 | 8 | 8 |
| | stat | n.s. | n.s. | n.s. | n.s. | n.s. | n.s. |

| | | | | | | | |
|---|---|---|---|---|---|---|---|
| n.a. not applicable; n.s. not significant: ** p < 0.05 vs. Orx + vehicle | | | | | | | |

### Measurement of Bone Mechanical Properties

### A) Compression Test of the Distal Femur

A segment of the distal femur was subject to a compression test. Unexpectedly, ORX animals displayed better bone strength than Sham-operated animals at Week 6. At Week 6, toremifene treatment resulted in bone strength similar to that of Sham-operated animals and estradiol treatment had the highest values in all groups. At Week 12, there was little difference between ORX and Sham-operated animals. Treatment with 5 mg/kg/d of toremifene maintained similar strength, while there was a small decrease in strength in the 10 mg/kg/d toremifene-treated group. Estradiol treatment had the highest values at Week 12, similar to Week 6. No statistically significant difference was evident in any of the treatment groups. Summarized data is shown in Tables 5a-5b.

**Table 5a: Compression Test of the Distal Femur Collected at Week 6**

| **Treatment Group** | **Time Point** | | **Maximum Load** | **Stiffness** | **Energy** | **Ultimate Strength** | **Elastic Modulus** | **Toughness** |
|---|---|---|---|---|---|---|---|---|
| | **weeks** | | **N** | **N/mm** | **mJ** | **N/mm²** | **MPa** | **MJ/m³** |
| Sham | 6 | mean | 457.46 | 3389.51 | 58.44 | 18.93 | 417.60 | 0.83 |
| vehicle | | SD | 71.82 | 935.44 | 16.28 | 3.52 | 135.35 | 0.29 |
| | | n | 8 | 8 | 8 | 8 | 8 | 8 |
| | | stat | n.s. | n.s. | n.s. | n.s. | ** | n.s. |
| ORX | 6 | mean | 537.44 | 4207.28 | 61.19 | 24.67 | 586.71 | 0.92 |
| vehicle | | SD | 136.73 | 916.23 | 24.77 | 8.34 | 158.48 | 0.45 |
| | | n | 8 | 8 | 8 | 8 | 8 | 8 |
| | | stat | n.a. | n.a. | n.a. | n.a. | n.a. | n.a. |
| ORX | 6 | mean | 469.64 | 4058.07 | 56.00 | 20.54 | 535.08 | 0.81 |
| 5 mg/kg/d | | SD | 104.65 | 717.34 | 19.13 | 5.87 | 114.01 | 0.31 |
| Toremifene | | n | 9 | 9 | 9 | 9 | 9 | 9 |
| | | stat | n.s. | n.s. | n.s. | n.s. | n.s. | n.s. |
| ORX | 6 | mean | 447.29 | 3549.70 | 66.19 | 18.47 | 440.77 | 0.91 |
| 10 mg/kg/d | | SD | 103.70 | 600.42 | 22.66 | 4.49 | 65.73 | 0.32 |
| Toremifene | | n | 9 | 9 | 9 | 9 | 9 | 9 |
| | | stat | n.s. | n.s. | n.s. | n.s. | n.s. | n.s. |
| ORX | 6 | mean | 561.45 | 4758.55 | 66.00 | 22.89 | 579.48 | 0.91 |
| 0.2 mg/kg/d | | SD | 107.68 | 688.47 | 18.57 | 5.41 | 126.28 | 0.30 |
| Estradiol | | n | 8 | 8 | 8 | 8 | 8 | 8 |
| | | stat | n.s. | n.s. | n.s. | n.s. | n.s. | n.s. |

| | | | | | | | | |
|---|---|---|---|---|---|---|---|---|
| n.a. not applicable; n.s. not significant; ** p < 0.05 vs. ORX + vehicle | | | | | | | | |

**Table 5b: Compression Test of the Distal Femur Collected at Week 12**

| **Treatment Group** | **Time Point** | | **Maximum Load** | **Stiffness** | **Energy** | **Ultimate Strength** | **Elastic Modulus** | **Toughness** |
|---|---|---|---|---|---|---|---|---|
| | **weeks** | | **N** | **N/mm** | **mJ** | **N/mm²** | **MPa** | **MJ/m³** |
| Sham | 12 | mean | 447.89 | 3804.83 | 54.10 | 18.18 | 469.64 | 0.73 |
| vehicle | | SD | 56.11 | 1063.77 | 21.97 | 2.75 | 146.22 | 0.28 |
| | | n | 6 | 6 | 6 | 6 | 6 | 6 |
| | | stat | n.s. | n.s. | n.s. | n.s. | n.s. | n.s. |
| ORX | 12 | mean | 469.46 | 3701.10 | 47.73 | 18.43 | 437.09 | 0.62 |
| vehicle | | SD | 131.36 | 1028.90 | 12.57 | 5.78 | 137.44 | 0.16 |
| | | n | 10 | 10 | 10 | 10 | 10 | 10 |
| | | stat | n.a. | n.a. | n.a. | n.a. | n.a. | n.a. |
| ORX | 12 | mean | 458.79 | 3175.07 | 53.13 | 18.09 | 379.32 | 0.69 |
| 5 mg/kg/d | | SD | 123.60 | 1367.19 | 17.22 | 4.76 | 164.22 | 0.20 |
| Toremifene | | n | 10 | 10 | 10 | 10 | 10 | 10 |
| | | stat | n.s. | n.s. | n.s. | n.s. | n.s. | n.s. |
| ORX | 12 | mean | 411.45 | 3366.63 | 45.57 | 16.81 | 418.05 | 0.62 |
| 10 mg/kg/d | | SD | 152.71 | 1438.59 | 14.91 | 6.06 | 193.28 | 0.19 |
| Toremifene | | n | 10 | 10 | 10 | 10 | 10 | 10 |
| | | stat | n.s. | n.s. | n.s. | n.s. | n.s. | n.s. |
| ORX | 12 | mean | 487.94 | 3714.71 | 55.94 | 20.66 | 473.02 | 0.78 |
| 0.2 mg/kg/d | | SD | 206.15 | 1521.39 | 19.74 | 8.82 | 189.11 | 0.29 |
| Estradiol | | n | 10 | 10 | 10 | 10 | 10 | 10 |
| | | stat | n.s. | n.s. | n.s. | n.s. | n.s. | n.s. |

| | | | | | | | | |
|---|---|---|---|---|---|---|---|---|
| n.a. not applicable; n.s. not significant, ** p < 0.05 vs. ORX + vehicle | | | | | | | | |

### B) Three Point Bending Test of the Femoral Shaft

There were no discernable differences in bone strength in any of the treatment groups at the femoral shaft. Summarized data is shown in Table 6.

**Table 6: Three Point Bending Test of the Femoral Shaft**

| **Treatment Group** | **Time Point** | | **Maximum Load** | **Stiffness** | **Energy** | **Ultimate Strength** | **Elastic Modulus** | **Toughness** |
|---|---|---|---|---|---|---|---|---|
| | **weeks** | | **N** | **N/mm** | **mJ** | **N/mm²** | **MPa** | **MJ/m³** |
| Sham | 12 | mean | 297.14 | 882.11 | 107.20 | 164.11 | 4006.27 | 7.13 |
| vehicle | | SD | 35.58 | 245.21 | 16.72 | 11.46 | 878.02 | 0.87 |
| | | n | 6 | 6 | 6 | 6 | 6 | 6 |
| | | stat | n.s. | n.s. | n.s. | n.s. | n.s. | n.s. |
| ORX | 12 | mean | 279.68 | 1048.31 | 90.07 | 149.74 | 4686.32 | 5.79 |
| vehicle | | SD | 21.99 | 61.93 | 11.96 | 12.80 | 586.19 | 0.72 |
| | | n | 10 | 10 | 10 | 10 | 10 | 10 |
| | | stat | n.a. | n.a. | n.a. | n.a. | n.a. | n.a. |
| ORX | 12 | mean | 263.54 | 944.78 | 83.40 | 150.45 | 4505.90 | 5.67 |
| 5 mg/kg/d | | SD | 17.77 | 188.45 | 14.61 | 11.21 | 713.56 | 1.06 |
| Toremifene | | n | 10 | 10 | 10 | 10 | 10 | 10 |
| | | stat | n.s. | n.s. | n.s. | n.s. | n.s. | n.s. |
| ORX | 12 | mean | 272.56 | 910.47 | 88.58 | 161.23 | 4557.46 | 6.16 |
| 10 mg/kg/d | | SD | 26.44 | 194.37 | 19.20 | 18.63 | 762.38 | 1.47 |
| Toremifene | | n | 10 | 10 | 10 | 10 | 10 | 10 |
| | | stat | n.s. | n.s. | n.s. | n.s. | n.s. | n.s. |
| ORX | 12 | mean | 287.22 | 986.71 | 98.61 | 172.51 | 5149.41 | 6.85 |
| 0.2 mg/kg/d | | SD | 35.41 | 87.54 | 17.64 | 13.74 | 691.15 | 0.89 |
| Estradiol | | n | 10 | 10 | 10 | 10 | 10 | 10 |
| | | stat | n.s. | n.s. | n.s. | ** | n.s. | n.s. |

| | | | | | | | | |
|---|---|---|---|---|---|---|---|---|
| n.a. not applicable; n.s. not significant; ** p < 0.05 vs. ORX + vehicle | | | | | | | | |

### C) Compression Test of the Vertebral Body

ORX induced decreases in bone strength, when compared to Sham-operated animals, toremifene treatment did not prevent these changes and, in fact, it may have worsened the changes at the 10 mg/kg/d dose. Estradiol treatment prevented loss of bone strength due to ORX. Summarized data is shown in Table 7.

**Table 7: Compression Test of the Vertebral Body**

| **Treatment Group** | **Time Point** | | **Maximum Load** | **Stiffness** | **Energy** | **Ultimate Strength** | **Elastic Modulus** | **Tonghness** |
|---|---|---|---|---|---|---|---|---|
| | **weeks** | | **N** | **N/mm** | **mJ** | **N/mm²** | **MPa** | **MJ/m³** |
| Sham | 12 | mean | 301.44 | 1280.09 | 65.62 | 24.54 | 419.72 | 1.33 |
| vehicle | | SD | 61.99 | 672.88 | 28.80 | 5.64 | 243.51 | 0.51 |
| | | n | 6 | 6 | 6 | 6 | 6 | 6 |
| | | stat | n.s. | n.s. | n.s. | n.s. | n.s. | n.s. |
| ORX | 12 | mean | 259.91 | 1429.91 | 51.70 | 22.29 | 484.39 | 1.11 |
| vehicle | | SD | 35.92 | 260.16 | 30.41 | 3.60 | 103.37 | 0.62 |
| | | n | 10 | 10 | 10 | 10 | 10 | 10 |
| | | stat | n.a. | n.a. | n.a. | n.a. | n.a. | n.a. |
| ORX | 12 | mean | 238.19 | 1391.70 | 36.60 | 19.08 | 446.38 | 0.72 |
| 5 mg/kg/d | | SD | 55.49 | 501.31 | 19.46 | 5.22 | 182.57 | 0.31 |
| Toremifene | | n | 10 | 10 | 10 | 10 | 10 | 10 |
| | | stat | n.s. | n.s. | n.s. | n.s. | n.s. | n.s. |
| ORX | 12 | mean | 206.48 | 1255.47 | 27.02 | 18.12 | 438.64 | 0.59 |
| 10 mg/kg/d | | SD | 61.98 | 477.48 | 9.95 | 5.56 | 161.47 | 0.22 |
| Toremifene | | n | 10 | 10 | 10 | 10 | 10 | 10 |
| | | stat | n.s. | n.s. | ** | n.s. | n.s. | ** |
| ORX | 12 | mean | 298.04 | 1648.17 | 43.60 | 25.75 | 589.03 | 0.94 |
| 0.2 mg/kg/d | | SD | 87.59 | 809.16 | 16.45 | 8.75 | 356.36 | 0.36 |
| Estradiol | | n | 10 | 10 | 10 | 10 | 10 | 10 |
| | | stat | n.s. | n.s. | n.s. | n.s. | n.s. | n.s. |

| | | | | | | | | |
|---|---|---|---|---|---|---|---|---|
| n.a. not applicable; n.s. not significant; ** p < 0.05 vs. ORX + vehicle | | | | | | | | |

### D) Cantilever Compression Test of the Femoral Neck

There were no changes in bone strength at the femoral neck in any of the treatment groups. Summarized data is shown in Table 8.

**Table 8: Cantilever Compression Test of the Femoral Neck**

| **Treatment Group** | **Time Point** | | **Maximum Load** | **Stiffness** | **Energy** |
|---|---|---|---|---|---|
| | **weeks** | | **N** | **N/mm** | **mJ** |
| Sham | 12 | mean | 113.50 | 198.48 | 50.67 |
| vehicle | | SD | 9.06 | 81.99 | 7.51 |
| | | n | 6 | 6 | 6 |
| | | stat | n.s. | n.s. | n.s. |
| ORX | 12 | mean | 117.83 | 204.06 | 46.50 |
| vehicle | | SD | 21.18 | 53.10 | 19.03 |
| | | n | 10 | 10 | 10 |
| | | stat | n.a. | n.a. | n.a. |
| ORX | 12 | mean | 116.46 | 211.77 | 46.53 |
| 5 mg/kg/d | | SD | 16.01 | 45.26 | 16.32 |
| Toremifene | | n | 10 | 10 | 10 |
| | | stat | n.s. | n.s. | n.s. |
| ORX | 12 | mean | 117.50 | 211.73 | 44.59 |
| 10 mg/kg/d | | SD | 18.16 | 52.67 | 13.90 |
| Toremifene | | n | 10 | 10 | 10 |
| | | stat | n.s. | n.s. | n.s. |
| ORX | 12 | mean | 114.96 | 209.95 | 40.07 |
| 0.2 mg/kg/d | | SD | 18.16 | 49.00 | 12.37 |
| Estradiol | | n | 10 | 10 | 10 |
| | | stat | n.s. | n.s. | n.s. |

| | | | | | |
|---|---|---|---|---|---|
| n.a. not applicable; n.s. not significant; ** p < 0.05 vs. ORX + vehicle | | | | | |

### SUMMARY

The effect of a SERM, toremifene, in preventing bone loss was tested in a model of male osteoporosis, orchidectomized rats. Two doses of toremifene, 5 and 10 mg/kg/d, and a comparator, estradiol at 0.2 mg/kg/d, were used to treat orchidectomized rats. Various skeletal sites were examined after 6 or 12 weeks of treatment. Bone mass, strength, microarchitecture by histomorphometry and ash density were used to determine changes in bone.

Orchidectomy induced a significantly elevated bone turnover state, as seen in increased osteoclasts and bone formation rate, at both the proximal tibia and lumbar vertebral body. As a result, there were reductions in bone mass at the distal femur, in trabecular bone volume at the proximal tibia and lumbar vertebral body and in strength parameters at the lumbar vertebral body, however, none of these changes reached statistically significant levels.

Toremifene at either 5 or 10 mg/kg/d significantly reduced the ochidectomy-induced elevation in bone turnover, with results similar to treatment with 0.2 mg/kg/d of estradiol. Whereas toremifene treatment was able to fully prevent the loss of trabecular bone volume, its effects on bone mass and strength were not as uniform. Treatment with 5 mg/kg/d of toremifene consistently exhibited similar or better values than vehicle-treated animals, but treatment with 10 mg/kg/d of toremifene displayed occasional reductions in bone mass and strength parameters. Estradiol treatment showed improved bone mass, microarchitecture and strength parameters at each of the bone sites tested.

In conclusion, toremifene treatment induced a significant reduction in orchidectomy-induced elevations of bone turnover. Toremifene performed well in preventing trabecular bone-loss, but its effect on cortical bone is less uniform. These findings demonstrate that torenufene can effectively treat male osteoporosis.

### EXAMPLE 2

### EFFECT OF TOREMIFENE ON HOT FLASHES

Due to the deleterious effect of testosterone on prostate cancer, the gold standard treatment for advanced disease is surgical or chemical castration of the patient However, the resulting low testosterone levels can have significant side effects including loss of bone leading to osteoporosis, hot flashes and gynecomastia. The adverse effect ofhot flashes is primarily a quality of life issue. However, hot flashes are often sited as the number one reason for the lack of compliance in these men.

In a phase II clinical trial intended to assess the effect of toremifene on bone mineral density (BMD) in men with advanced prostate cancer on luteinizing hormone releasing hormone agonists (LHRHa), an assessment of hot flash frequency was included as a secondary endpoint. The study enrolled a total of 46 subjects that had been on LHRHa for at least 12 months. The treatment period of the study was 6 months. The results from subjects with hot flash frequency assessments at baseline and after 6 months of treatment are included in Table 9.

**Table 9: FREQUENCY OF HOT FLASHES - # of subjects (% subjects)**

| Dose | Increase | No Change | Decrease |
|---|---|---|---|
| Placebo (n=11) | 6 (55%) | 4 (36%) | 1 (9%) |
| 20 mg (n=10) | 3 (30%) | 4 (40%) | 3 (30%) |
| 40 mg (n=10) | 3 (30%) | 5 (50%) | 2 (20%) |
| 60 mg (n=8) | 1 (13%) | 6 (75%) | 1 (13%) |

Thus, that there is a decrease in the percentage of subjects that experience an increase in hot flash frequency with increasing toremifene dose. These findings demonstrate that SERMs such as toremifene can effectively treat hot flashes.

### EXAMPLE 3

### TOREMIFENE REVERSES ADT-INDUCED OSTEOPOROSIS

### MATERIALS AND METHODS

46 Consenting patients with prostate cancer who had been on ADT for at least 12 months were randomly assigned to receive placebo or 20 mg, 40 mg, or 60 mg of toremifene citrate (Acapodene™), administered orally once daily for 6 months. Assessments included bone mineral density (BMD) determination by dual energy X-ray absorptiometry (DEXA), bone turnover markers, bone resorption markers. BMD was assessed by DXA. Levels of bone turnover markers were assessed.

Based on reports in the literature, the anti-androgen bicalutamide can alter bone metabolism. To avoid potential confounding effects from bicalutamide, patients receiving bicalutamide were excluded from the analysis.

### RESULTS

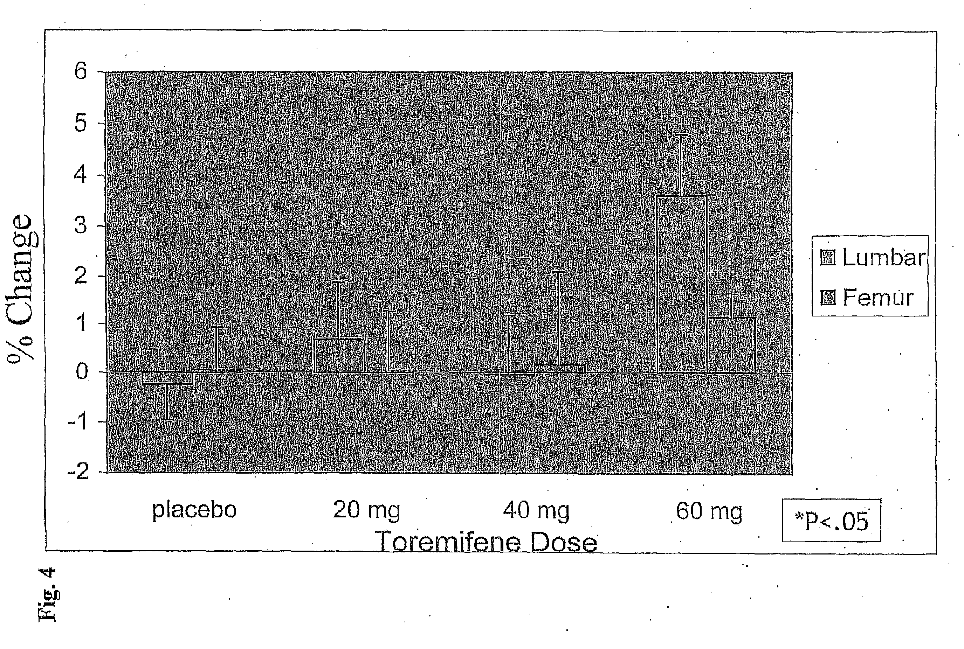

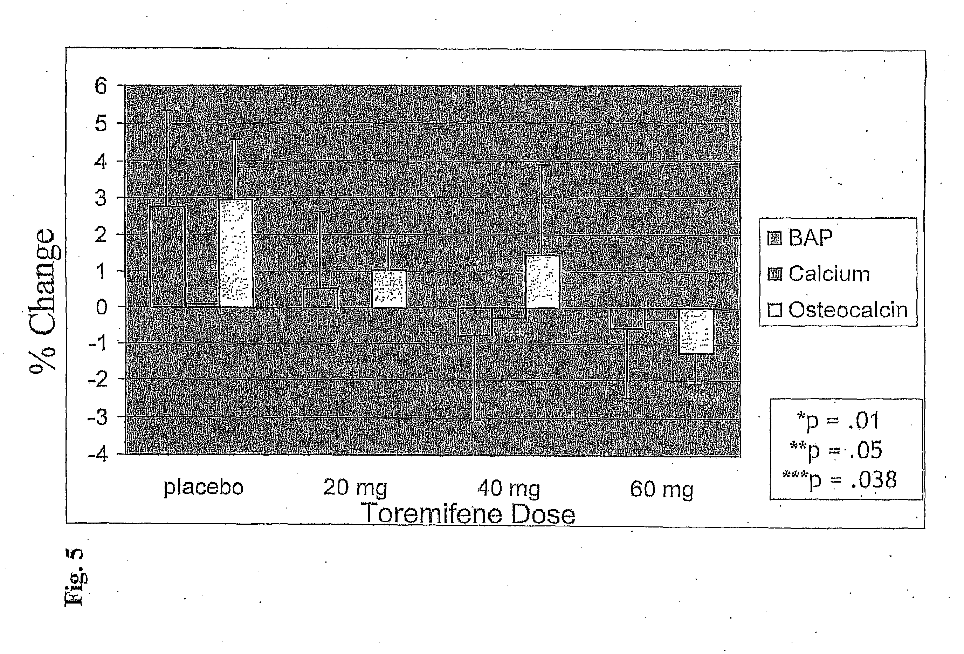

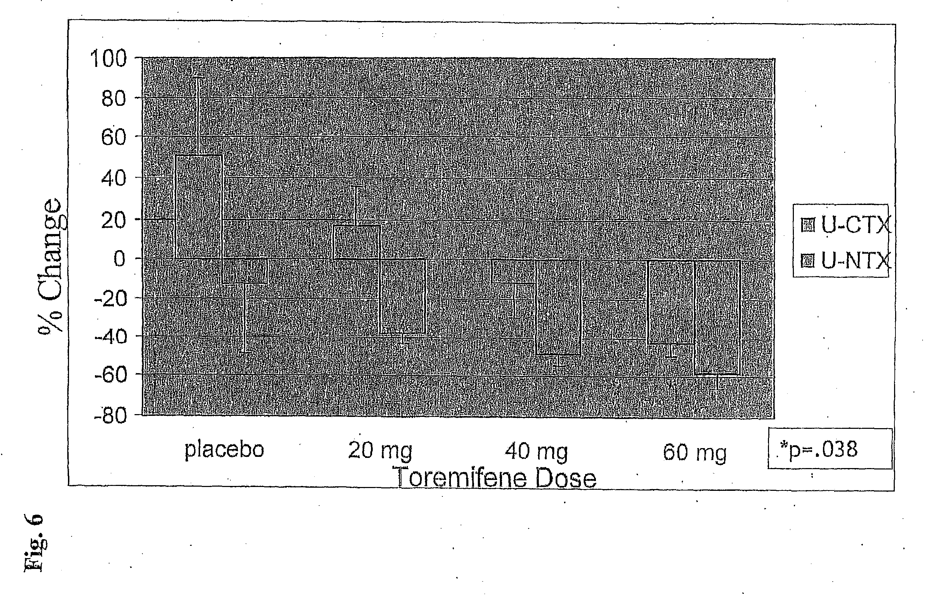

Toremifene was well tolerated at all doses studied. Administration of 60 mg/day of toremifene resulted in a statistically significant increase of BMD after 6 months of therapy compared to a decrease in the placebo group (p < 0.05; Figure 4); while addition of 20 mg/day or 40 mg/day resulted in a smaller increase. In addition, the bone turnover markers bone alkaline phosphatase (BAP), calcium, and osteocalcium were also reduced significantly as a result of 40 mg/day or 60 mg/day toremifene treatment (Figure 5), showing that bone turnover was decreased. Furthermore, the bone resorption markers Urinary C Telopeptide (U-CTX) and Urinary N Telopeptide (U-NTX) were reduced significantly as a result of toremifene treatment (Figure 6), showing that bone resorption was decreased.

These findings demonstrate that anti-estrogens such as toremifene positively affect BMD and reduces bone turnover and bone resorption in patients receiving ADT. These findings also show that anti-estrogens such as toremifene can halt and reverse osteoporosis, decreased BMD, and increased bone turnover and bone resorption that result from ADT.

### EXAMPLE 4

### TOREMIFENE REDUCES FSH LEVELS IN PATIENTS UNDERGOING

### ADT

### MATERIALS AND METHODS

### LH and FSH assays

LH (Luteinizing hormone) and FSH (Follicle stimulating hormone) were measured in serum by the national Hormone and Peptide Program (Dr. AF Parlow, UCLA, CA) using RIA (radioimmunoassay) kits.

### RESULTS

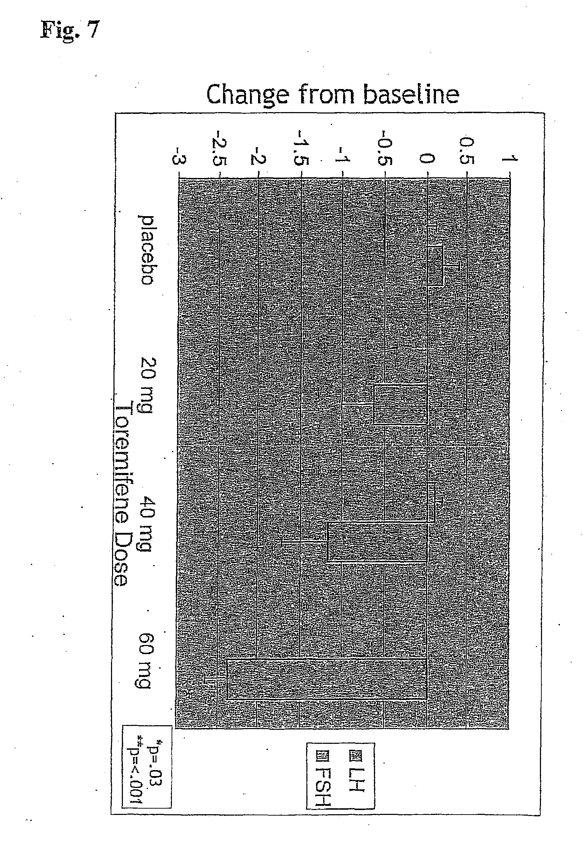

Serum levels of the pituitary hormones LH and FSH were measured in the subjects of Example 1. Subjects treated with toremifene exhibited feedback inhibition of the hypothalamus-pituitary axis, as evidenced by a dose dependent reduction in serum FSH (Figure 7).

These findings provide further evidence that anti-estrogens such as toremifene prevent hot flashes and gynecomastia in subjects undergoing ADT. The effect of toremifene on FSH levels is very likely to mediate a positive effect on hair loss as well.

## Claims

1. Toremifene or its analog, derivative, isomer, metabolite, pharmaceutically acceptable salt, pharmaceutical product, hydrate, N-oxide, or any combination thereof for treating, reducing the incidence of, suppressing, or inhibiting androgen-deprivation therapy induced gynecomastia in a subject.

2. Toremifene according to claim 1, wherein said toremifene is toremifene citrate.

3. Toremifene according to claim 1, wherein said subject is human.

4. Toremifene according to claim 3, wherein said human subject is male.

5. Toremifene according to any one of the preceding claims, wherein said subject suffers from prostate cancer.

6. Use of toremifene or its analog, derivative, isomer, metabolite, pharmaceutically acceptable salt, pharmaceutical product, hydrate, N-oxide, or any combination in the preparation of a composition for treating, reducing the incidence of, suppressing, or inhibiting androgen-deprivation therapy induced gynecomastia in a subject.

7. The use according to claim 6, wherein said composition comprises a pharmaceutically acceptable carrier.

8. The use according to claim 6, wherein said composition is prepared in liquid form for intravenous, intraarterial, or intramuscular injection, in pellet form for subcutaneous implantation, in a liquid or solid form for oral administration, or for topical application.

9. The use according to claim 8, wherein said composition is a pellet, a tablet, a capsule, a solution, a suspension, an emulsion, an elixir, a gel, a cream, a suppository or a parenteral formulation.
